(19) Europäisches Patentamt
European Patent Office
Office européen des brevets

(11) Veröffentlichungsnummer: **0 139 947**
**B1**

(12) # EUROPÄISCHE PATENTSCHRIFT

(45) Veröffentlichungstag der Patentschrift: **10.08.88**

(21) Anmeldenummer: **84109646.4**

(22) Anmeldetag: **14.08.84**

(51) Int. Cl.⁴: **C 07 D 239/42**, C 07 D 239/47, C 07 D 239/52, C 07 D 239/46, C 07 D 295/14, C 07 D 295/08, C 07 D 251/16, C 07 D 251/46, C 07 D 251/50, C 07 C 143/833 // A01N47/36, A01N47/30

(54) **2-Amino-alkenylsulfonylharnstoffe.**

(30) Priorität: **25.08.83 DE 3330603**

(43) Veröffentlichungstag der Anmeldung: **08.05.85 Patentblatt 85/19**

(45) Bekanntmachung des Hinweises auf die Patenterteilung: **10.08.88 Patentblatt 88/32**

(84) Benannte Vertragsstaaten: **AT BE CH DE FR GB IT LI NL SE**

(56) Entgegenhaltungen:
**EP-A-0 071 958**
**DD-A-22 101**
**DD-A-115 491**
**DE-A-3 300 569**
**US-A-4 371 391**

(73) Patentinhaber: **HOECHST AKTIENGESELLSCHAFT, Postfach 80 03 20, D-6230 Frankfurt am Main 80 (DE)**

(72) Erfinder: **Willms, Lothar, Dr., Grüner Weg 4, D-5463 Unkel (DE)**

**EP 0 139 947 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Es ist bekannt, daß man Chlorsulfonylharnstoffe mit primären und sekundären Aminen unter HCl-Abspaltung zu Aminosulfonylharnstoffen umsetzen kann (vgl. R. Graf, Angew. Chemie, Int. Ed. Engl. 7, 172 (1968); DE-PS 940 292, Gl. 1).

Überraschenderweise wurde nun gefunden, daß man bei der Umsetzung von Halogensulfonylharnstoffen mit Enaminen, welche in β-Stellung zur Aminogruppe ein Wasserstoffatom tragen, in hohen Ausbeuten 1,2-ungesättigte Alkenylsulfonylharnstoffe erhält. Auf diese Weise lassen sich neue 2-Amino-alkenylsulfonylharnstoffe herstellen.

Gegenstand der vorliegenden Erfindung sind daher neue 2-Amino-alkenylsulfonylharnstoffe der Formel (I)

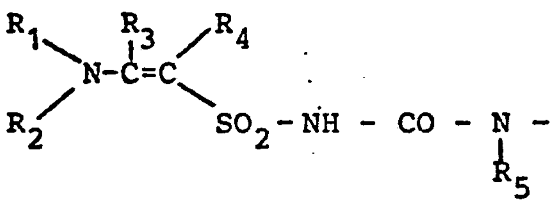

(I)

worin

$R_1$ und $R_2$ unabhängig voneinander Wasserstoff $(C_1-C_8)$-Alkyl, $(C_3-C_7)$-Cycloalkyl, Phenyl, welches gegebenenfalls ein- oder mehrfach durch Halogen, $(C_1-C_4)$-Alkyl, $(C_1-C_4)$-Alkoxy, $(C_1-C_4)$-Alkylthio, $(C_1-C_4)$-Alkoxycarbonyl, $CF_3$, $NO_2$ oder $CH_3SO_2$-substituiert sein kann, bedeuten oder die Reste $R_1$ und $R_2$ zusammen mit dem gemeinsamen Stickstoffatom einen 5- bis 7-gliedrigen aliphatischen Ring bilden, worin eine $CH_2$ Gruppe gegebenenfalls durch Sauerstoff, NH oder $N(C_1-C_4)$-Alkyl ersetzt sein kann,

$R_3$ Wasserstoff, $(C_3-C_7)$Cycloalkyl, $(C_1-C_6)$Alkyl, $(C_1-C_4)$Alkoxy, $(C_2-C_4)$Alkenyloxy, $(C_2-C_4)$Alkinyloxy, $(C_1-C_4)$Alkylsulfenyl, $(C_1-C_4)$Alkylsulfinyl, $(C_1-C_4)$Alkyl-sulfonyl, $-SO_2NH(C_1-C_4)$Alkyl, $-SO_2N[(C_1-C_4)Alkyl]_2$, einen Phenylrest, der gegebenenfalls ein oder mehrfach durch Halogen, $CF_3$, $(C_1-C_4)$Alkyl, $(C_1-C_4)$Alkoxy, $(C_1-C_4)$Alkoxycarbonyl, $(C_1-C_4)$-Alkylsulfenyl, $(C_1-C_4)$-Alkylsulfinyl oder $(C_1-C_4)$-Alkylsulfonyl substituiert ist, Benzyl, $(C_1-C_4)$Alkoxycarbonyl, $(C_2-C_4)$Alkenyloxy-carbonyl, $(C_2-C_4)$Alkinyloxycarbonyl, Aminocarbonyl, $(C_1-C_4)$Alkylaminocarbonyl oder$[(C_1-C_4)Alkyl]_2$aminocarbonyl,

$R_4$ die Bedeutung von $R_3$ umfaßt und zusätzlich Formyl, $(C_1-C_6)$-Alkanoyl, Benzoyl, das gegebenenfalls durch Halogen, $(C_1-C_4)$Alkyl, $(C_1-C_4)$Alkoxy, $(C_1-C_4)$Alkylthio, $CF_3$, $NO_2$ oder $(C_1-C_4)$Alkoxycarbonyl substituiert sein kann, $(C_3-C_7)$Cycloalkylcarbonyl, $C_6H_5-CH_2-O-CO-$, bedeuten oder $R_3$ und $R_4$ gemeinsam mit den sie verbindenden olefinischen Kohlenstoffatomen ein 5- bis 8-gliedriges aliphatisches Ringsystem bilden,

$R_5$ Wasserstoff, $(C_1-C_6)$-Alkyl, $(C_1-C_4)$-Alkenyl, $(C_1-C_4)$-Alkinyl, $(C_1-C_4)$-Alkoxy oder $(C_1-C_4)$-Alkoxycarbonyl,

$R_6$ $(C_1-C_8)$-Alkyl oder $(C_3-C_7)$-Cycloalkyl, Phenyl, welches gegebenenfalls ein oder mehrfach durch Halogen $(C_1-C_4)$-Alkyl, $(C_1-C_4)$-Alkoxy, $(C_1-C_4)$-Alkylthio, $CF_3$, $NO_2$ oder $(C_1-C_4)$-Alkoxycarbonyl substituiert sein kann oder den Rest

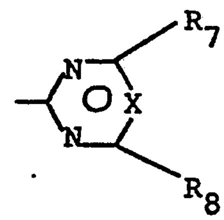

bedeutet, worin

$R_7$ und $R_8$ unabhängig voneinander Wasserstoff, $(C_1-C_4)$-Alkyl oder $(C_1-C_4)$-Alkoxy, welche gegebenenfalls ein- bis zweifach durch $(C_1-C_2)$-Alkoxy oder ein- bis dreifach durch Halogen substituiert sind, Halogen, $-(CHR_9)_m-S(O)_nR_{10}$, $NH_2$, $NH(C_1-C_4)$-Alkyl $N[(C_1-C_4)\text{-Alkyl}]_2$, $(C_2-C_4)$-Alkenyl, $(C_2-C_4)$-Alkinyl, $(C_2-C_4)$-Alkenyloxy oder $(C_2-C_4)$-Alkinyloxy,

$R_9$ Wasserstoff oder $(C_1-C_4)$-Alkyl,

$R_{10}$ $(C_1-C_4)$-Alkyl oder Benzyl,

X CH, C-Hal mit Hal = Chlor oder Brom, C-$(C_1-C_4)$-Alkyl, C-$(C_1-C_4)$-Alkoxy oder N,

m eine Zahl von null bis drei und

n eine Zahl von null bis zwei

bedeuten.

Gegenstand der vorliegenden Erfindung ist weiterhin ein Verfahren zur Herstellung der Verbindungen der Formel (I), dadurch gekennzeichnet, daß man Verbindungen der Formel (II) worin X' = Chlor oder Fluor bedeutet und $R_5$, $R_6$ die obengenannten Bedeutungen besitzen,

$$X' - SO_2 - NH - CO - \underset{\displaystyle R_5}{\underset{|}{N}} - R_6 \qquad (II)$$

mit Enaminen der Formel (III), worin

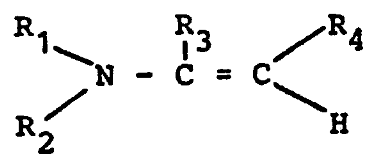

(III)

$R_1$ $R_2$, $R_3$ und $R_4$ die obengenannten Bedeutungen besitzen, umsetzt.

Überraschenderweise liefert dieses Verfahren die gewünschten Verbindungen der Formel I in hohen Ausbeuten, obwohl die Enamine der Formel III über verschiedene nucleophile Zentren verfügen und daher prinzipiell bei der Umsetzung mit Elektrophilen wie den Verbindungen der Formel II in verschiedener Weise reagieren sollten (vgl. G. Opitz Angew. Chem. 79, 171 (1967).

Die Verbindungen der Formel II sind bekannt

oder nach literaturbekannten Verfahren zugänglich. Sie werden beispielsweise durch Addition von Chlorsulfonylisocyanat an primäre oder sekundäre Aniline oder Aminoheterocyclen erhalten (vgl. R. Graf, Angew. Chem., Int. Ed. Engl 7, 172 (1968); Europäische Patentanmeldung 45 196).

Die Ausgangsverbindungen der Formel (III) werden nach literaturbekannten Verfahren (s. z. B. "Enamines-Synthesis, Structure and Reactions", Hrsg. A.G. Cook, M. Dekker Inc., N.Y./London 1969) hergestellt.

Die Umsetzung der Verbindungen (II) und (III) erfolgt vorzugsweise in inerten aprotischen Lösungsmitteln wie beispielsweise Acetonitril, halogenierten Aliphaten wie Dichlormethan, Kohlenwasserstoffen wie Toluol, Xylol oder Heterocyclen wie Tetrahydrofuran oder Dioxan bei Temperaturen zwischen -78° C und der Siedetemperatur des Lösungsmittels. Das Molverhältnis der Reaktionskomponenten ist nicht kritisch und kann bei geeigneter Versuchsführung innerhalb weiter Grenzen variiert werden.

Die Synthese der Verbindungen der Formel I wird vorzugsweise in Gegenwart eines Säureakzeptors durchgeführt. Als solche kommen in Frage: anorganische Verbindungen wie z. B. Alkali- oder Erdalkalicarbonate, -hydrogencarbonate, -oxide oder -hydroxide oder organische Basen wie aliphatische oder cycloaliphatische tertiäre Amine wie z. B. Triethylamin oder N,N-Diisopropylamin oder stickstoffhaltige Aromaten wie z. B. Pyridin oder 2,6-Lutidin. Als Hilfsbase kann auch die Enaminkomponente (III) dienen, die dann im Überschuß, vorzugsweise in einem Molverhältnis III : II von 1,8 bis 2,4 : 1, eingesetzt wird.

Die erfindungsgemäßen 2-Amino-alkenylsulfonylharnstoffe der allgemeinen Formel (I) stellen interessante Vorprodukte zur Synthese von neuen Pflanzenschutzmitteln und Pharmazeutika dar.

So werden z. B. bei der Umsetzung von (I) mit 1,2-Dinucleophilen heterocyclische Sulfonylharnstoffe mit herbiziden Eigenschaften gewonnen, s. EP-A-142 629.

Die erfindungsgemäßen Verbindungen der Formel (I) weisen ferner zum Teil selbst herbizide und pflanzenwachstumsregulierende Wirksamkeiten auf.

Die nachfolgenden Beispiele dienen zur Erläuterung der Erfindung.

**Beispiel 1**

Ethyl-2-[3-(4,6-dimethyl-2-pyrimidinyl)-ureidosulfonyl]-3-methylamino-2-butenoat

9,2 ml (0,105 Mol) CSI (CSI = chlorsulfonylisocyanat) wurden in 250 ml wasserfreiem Methylenchlorid bei -70° C unter Stickstoff in ca. 5 Minuten mit 12,7 g (0,1 Mol) 2-Amino-4,6-dimethylpyrimidin versetzt. Man ließ das Reaktionsgemisch in ca. 1 h auf 0° C erwärmen, kühlte es dann auf -70° C ab und versetzte es in 1 h mit 28,6 g (0,2 Mol) ß-Methylaminocrotonsäureethylester (gelöst in ca. 50 ml wasserfreiem Methylenchlorid). Man ließ das Reaktionsgemisch auf Raumtemperatur erwärmen, rührte 18 h nach, extrahierte viermal mit Wasser und fällte das Produkt mit n-Hexan aus.

Ausbeute: 32,6 g (88 % d.Th.). Fp. 177-180° C.

**Beispiel 2**

N-[(4,6-Dimethylpyrimidin-2-yl)aminocarbonyl]-1-methyl-carbonyl-2-methylamino-prop-1-en-1-yl-sulfonamid

9,2 ml CSI (0,105 Mol) wurden in 250 ml absolutem Methylenchlorid bei -70° C unter Stickstoffatmosphäre in ca. 5 min mit 12,3 g (0,1 Mol) 2-Amino-4,6-dimethylpyrimidin versetzt. Man ließ das Reaktionsgemisch innerhalb von 1 h auf 0° C erwärmen, kühlte auf -70° C und tropfte nacheinander 11,4 g (0,1 Mol) 4-Methylamino-but-3-en-2-on (gelöst in 50 ml $CH_2Cl_2$) und 10,1 g (0,1 Mol) $Et_3N$ hinzu. Man ließ das Reaktionsgemisch in ca. 4 h auf Raumtemperatur erwärmen, rührte 18 h bei Raumtemperatur, extrahierte viermal mit je 150 ml Wasser und trocknete über Natriumsulfat. Anschließend wurde das Gemisch bei 0° C tropfenweise mit n-Hexan versetzt. Das ausgefallene Produkt wurde abgesaugt und im Vakuum getrocknet. Fp. 192° C

Ausbeute: 29,3 g (86 % d.Th.)

**Beispiel 3**

Methyl-2-[3-(4,6-dimethyl-2-pyrimidinyl)-ureidosulfonyl]-3-amino-2-butenoat

9,2 ml (0,105 Mol) CSI wurden bei -70° C in 250 ml wasserfreiem Methylenchlorid unter Stickstoffatmosphäre in ca. 5 min. mit 12,3 g (0,1 Mol) 2-Amino-4,6-dimethylpyrimidin versetzt. Man ließ das Reaktionsgemisch in 1 h auf 0° C erwärmen, kühlte dann auf -70° C und tropfte in 2 h 23,0 g (0,2 Mol) ß-Aminocrotonsäuremethylester (gelöst in 100 ml wasserfreiem Methylenchlorid) hinzu. Nach 18 h Nachrühren bei Raumtemperatur wurde viermal mit Wasser extrahiert und der unlösliche Rückstand abgesaugt.

Man erhielt 24,6 g (72 % d.Th.) des gewünschten Produktes mit Fp. 222 - 225° C

Aus dem Methylenchlorid-Filtrat wurden nach dem Trocknen über Natriumsulfat und Zugabe von n-Hexan 7,0 (20,4 % d.Th.) Methyl-3-[(3-(4,6-dimethyl-2-pyrimidinyl)ureidosulfonyl-amino]-2-butenoat als Nebenprodukt isoliert.

Fp. 139-140° C (Z.)

Auf gleiche Weise wurden die in Tabelle 1 aufgeführten Sulfonylharnstoffderivate der Formel I hergestellt.

Tabelle 1

| Verb.-Nr. | $R_1$ | $R_2$ | $R_3$ | $R_4$ | $R_5$ | $R_6$ | Schmp. (°C) |
|---|---|---|---|---|---|---|---|
| 4 | H | H | $CH_3$ | $COOC_2H_5$ | H | 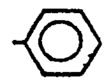 | 159-160 (Z) |
| 5 | H | H | $CH_3$ | $COOCH_3$ | H | 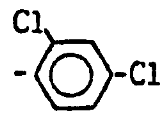 | |
| 6 | $CH_3$ | $CH_3$ | 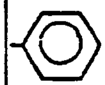 | $COOCH_3$ | H | 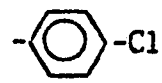 | |
| 7 | 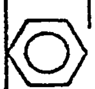 | H | 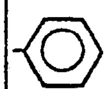 | $COOC_2H_5$ | $CH_3$ | 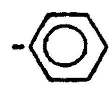 | |
| 8 | $-(CH_2)_4-$ | | $CH_3$ | $COOCH_3$ | H | 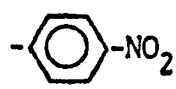 | |
| 9 | $C_2H_5$ | H | $CH_3$ | $COOCH_3$ | H | 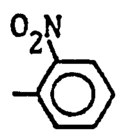 | |

Tabelle 1 (Fortsetzung)

| Verb.-Nr. | $R_1$ | $R_2$ | $R_3$ | $R_4$ | $R_5$ | $R_6$ | Schmp. |
|---|---|---|---|---|---|---|---|
| 10 | $nC_3H_7$ | H | $CH_3$ | $COOCH_3$ | H | 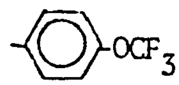 | |
| 11 | $CH_3$ | H | $CH_3$ | CO | H | 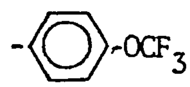 | |
| 12 | $CH_3$ | $CH_3$ | $C_2H_5$ | $COOCH_3$ | H | 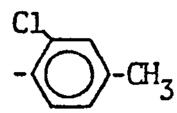 | |
| 13 | $-(CH_2)_2-O-(CH_2)_2$ | | $-(CH_2)_4-$ | | H | 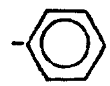 | |
| 14 | $-(CH_2)_2-O-(CH_2)_2-$ | | $-(CH_2)_3-$ | | H | 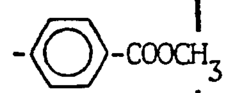 | |
| 15 | $(CH_2)_2N(CH_3)-(CH_2)_2-$ | | $-(CH_2)_4-$ | | H | 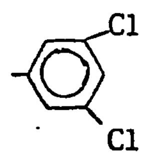 | |
| 16 | 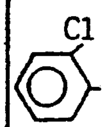 | H | H | $COOC_2H_5$ | H | 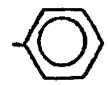 | |
| 17 | $-(CH_2)_2-O-(CH_2)_2-$ | | H | 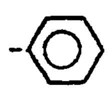 | H | 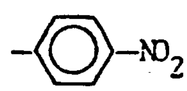 | |
| 18 | 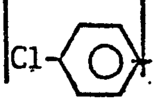 | H | $CH_3$ | $COCH_3$ | H | 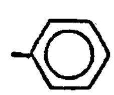 | |

Tabelle 1 (Fortsetzung)

| Verb.-Nr. | $R_1$ | $R_2$ | $R_3$ | $R_4$ | $R_5$ | $R_6$ | Schmp. (°C) |
|---|---|---|---|---|---|---|---|
| 19 | $CH_3$ | H | $CH_3$ | $COOC_2H_5$ | H | 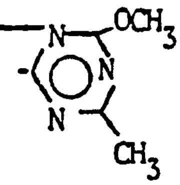 | 168 |
| 20 | $CH_3$ | $CH_3$ | $CH_3$ | $COOC_2H_5$ | H | 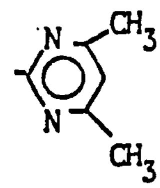 | 161 |
| 21 | $CH_3$ | $CH_3$ | $CH_3$ | $COOC_2H_5$ | H | 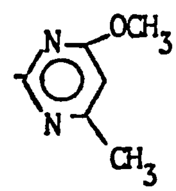 | 159-161 |
| 22 | H | H | $CH_3$ | $COOC_2H_5$ | H | 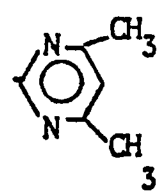 | 180 |
| 23 | $CH_3$ | H | $CH_3$ | $COOCH_3$ | H | 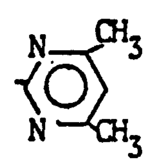 | 175 |
| 24 | $CH_3$ | H | $CH_3$ | $COOC_2H_5$ | H | 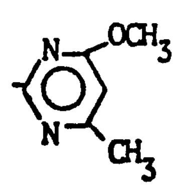 | 169-171 |

Tabelle 1 (Fortsetzung)

| Verb.-Nr. | $R_1$ | $R_2$ | $R_3$ | $R_4$ | $R_5$ | $R_6$ | Schmp. |
|---|---|---|---|---|---|---|---|
| 25 | $CH_3$ | H | $CH_3$ | $COOC_2H_5$ | H | 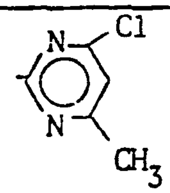 | 169-171 |
| 26 | $CH_3$ | H | $CH_3$ | $COOC_2H_5$ | H | 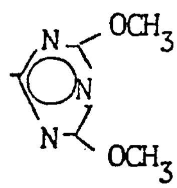 | 190 |
| 27 | 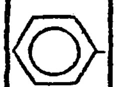 | H | $CH_3$ | $COOC_2H_5$ | H | 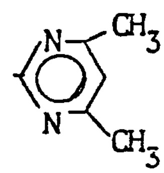 | 114 |
| 28 | $CH_3$ | H | $CH_3$ | $COOC_2H_5$ | $C_2H_5$ | 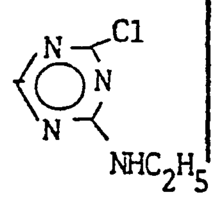 | 147 |
| 29 | $CH_3$ | $CH_3$ | $CH_3$ | $COOC(CH_3)_3$ | H | 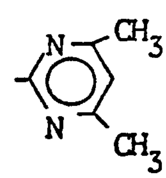 | 140-143 |
| 30 | $CH_3$ | H | $CH_3$ | $COOC_2H_5$ | H | 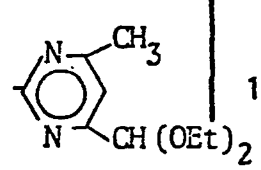 | 136-148 |

Tabelle 1 (Fortsetzung)

| Verb.-Nr. | $R_1$ | $R_2$ | $R_3$ | $R_4$ | $R_5$ | $R_6$ | Schmp. |
|---|---|---|---|---|---|---|---|
| 31 | $-(CH_2)_2-O-(CH_2)_2-$ | | $-(CH_2)_4-$ | | H | 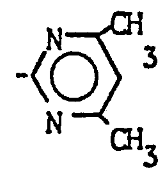 | Harz |
| 32 | $CH_3$ | H | $CH_3$ | $COCH_3$ | H | 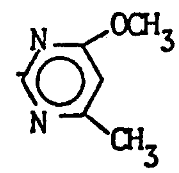 | 159-161 |
| 33 | $CH_3$ | H | $CH_3$ | $COCH_3$ | H | 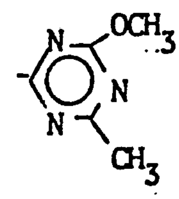 | 156 |
| 34 | H | H | $CH_3$ | $COCH_3$ | H | 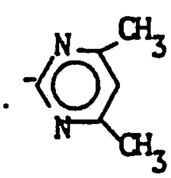 | 210-212 |
| 35 | 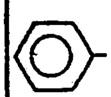 | H | $CH_3$ | $COCH_3$ | H | 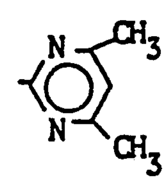 | 149-151 |
| 36 | 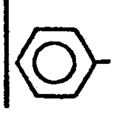 | H | 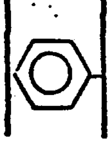 | $COCH_3$ | H | 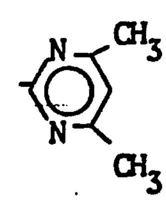 | 157-159 |

Tabelle 1 (Fortsetzung)

| Verb.-Nr. | $R_1$ | $R_2$ | $R_3$ | $R_4$ | $R_5$ | $R_6$ | Schmp. |
|---|---|---|---|---|---|---|---|
| 37 | $CH_3$ | H | 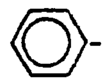 | $COCH_3$ | H | 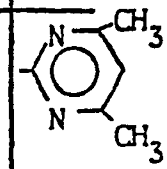 | 146-148 |
| 38 | $CH_3$ | H | $CH_3$ | $COCH_3$ | H | 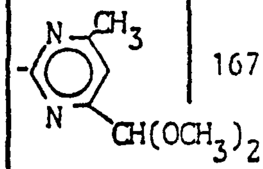 | 167-169 |
| 39 | $CH_3$ | H | 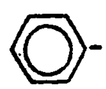 | CO-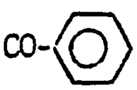 | H | 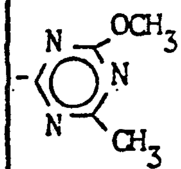 | |
| 40 | $CH_3$ | $CH_3$ | $CH_3$ | $COOCH_2$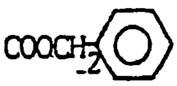 | H | 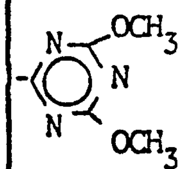 | |
| 41 | $-(CH_2)_4-$ | | $CH_3$ | $COCH_3$ | H | 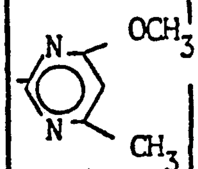 | |
| 42 | $-(CH_2)_2-O-(CH_2)_2-$ | | $CH_3$ | $COCH_3$ | H | 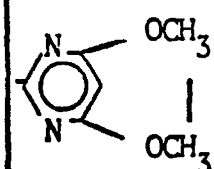 | |
| 43 | $CH_3$ | $CH_3$ | $C_2H_5$ | $COC_2H_5$ | H | 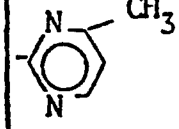 | |
| 44 | $CH_3$ | H | $CH_3$ | $COCH_3$ | H | 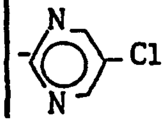 | |

Tabelle 1 (Fortsetzung)

| Verb.-Nr. | $R_1$ | $R_2$ | $R_3$ | $R_4$ | $R_5$ | $R_6$ | Schmp. |
|---|---|---|---|---|---|---|---|
| 45 | $CH_3$ | H | $CH_3$ | $COCH_3$ | H | 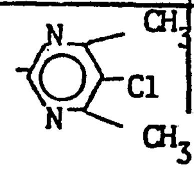 | |
| 46 | H | H | $CCl_3$ | $COOC_2H_5$ | H | 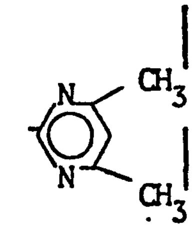 | |

**Patentansprüche** für die Vertragsstaaten BE CH DE FR GB IT LI NL SE

1. Verbindungen der Formel I, worin

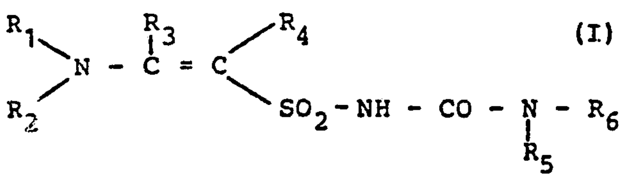
(I)

$R_1$ und $R_2$ unabhängig voneinander Wasserstoff, $(C_1-C_8)$-Alkyl, $(C_3-C_7)$-Cycloalkyl, Phenyl, welches gegebenenfalls ein- oder mehrfach durch Halogen, $(C_1-C_4)$-Alkyl, $(C_1-C_4)$-Alkoxy, $(C_1-C_4)$-Alkyl-thio, $(C_1-C_4)$-Alkoxycarbonyl, $CF_3$, $NO_2$ oder $CH_3SO_2$-substituiert sein kann, bedeuten oder die Reste $R_1$ und $R_2$ zusammen mit dem gemeinsamen Stickstoffatom einen 5- bis 7-gliedrigen aliphatischen Ring bilden, worin eine $CH_2$-Gruppe gegebenenfalls durch Sauerstoff, NH oder $N(C_1-C_4)$-Alkyl ersetzt sein kann,

$R_3$ Wasserstoff, $(C_3-C_7)$Cycloalkyl, $(C_1-C_6)$Alkyl, $(C_1-C_4)$-Alkoxy, $(C_2-C_4)$Alkenyloxy, $(C_2-C_4)$Alkinyloxy, $(C_1-C_4)$Alkylsulfenyl, $(C_1-C_4)$Alkylsulfinyl, $(C_1-C_4)$-Alkylsulfonyl, $-SO_2NH(C_1-C_4)$Alkyl, $-SO_2N[(C_1-C_4)\text{-Alkyl}]_2$, einen Phenylrest, der gegebenenfalls ein- oder mehrfach durch Halogen, $CF_3$, $(C_1-C_4)$Alkyl, $(C_1-C_4)$Alkoxy, $(C_1-C_4)$Alkoxycarbonyl, $(C_1-C_4)$Alkylsulfenyl, $(C_1-C_4)$Alkylsulfinyl oder $(C_1-C_4)$Alkylsul-fonyl substituiert ist, Benzyl, $(C_1-C_4)$Alkoxycarbonyl, $(C_2-C_4)$Alkenyloxycarbonyl, $(C_2-C_4)$Alkinyloxycarbonyl, Aminocarbonyl, $(C_1-C_4)$Alkylaminocarbonyl oder $[(C_1-C_4)\text{Alkyl}]_2$aminocarbonyl,

$R_4$ die Bedeutung von $R_3$ umfaßt und zusätzlich Formyl, $(C_1-C_6)$-Alkanoyl, Benzoyl, das gegebenenfalls durch Halogen, $(C_1-C_4)$Alkyl, $(C_1-C_4)$Alkoxy, $(C_1-C_4)$Alkylthio, $CF_3$, $NO_2$ oder $(C_1-C_4)$Alkoxycarbonyl substituiert sein kann, $(C_3-C_7)$Cycloalkylcarbonyl, $C_6H_5-CH_2-O-CO-$, bedeuten oder $R_3$ und $R_4$ gemeinsam mit den sie verbindenden olefinischen Kohlenstoffatomen ein 5- bis 8-gliedriges cycloaliphatisches Ringsystem bilden,

$R_5$ Wasserstoff, $(C_1-C_6)$-Alkyl, $(C_1-C_4)$-Alkenyl, $(C_1-C_4)$-Alkinyl, $(C_1-C_4)$-Alkoxy oder $(C_1-C_4)$-Alkoxycarbonyl bedeutet,

$R_6$ $(C_1-C_8)$-Alkyl oder $(C_3-C_7)$-Cycloalkyl, Phenyl, welches gegebenenfalls ein oder mehrfach durch Halogen, $(C_1-C_4)$-Alkyl, $(C_1-C_4)$-Alkoxy, $(C_1-C_4)$-Alkylthio, $CF_3$, $NO_2$ oder $(C_1-C_4)$-Alkoxycarbonyl substituiert sein kann oder den Rest

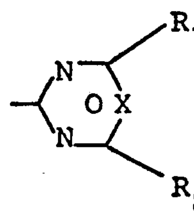

bedeutet, worin

$R_7$ und $R_8$ unabhängig voneinander Wasserstoff, $(C_1-C_4)$-Alkyl oder $(C_1-C_4)$-Alkoxy, welche gegebenenfalls ein- bis zweifach durch $(C_1-C_2)$-Alkoxy oder ein- bis dreifach durch Halogen substituiert sind, Halogen, $-(CHR_9)_m-S(O)_nR_{10}$, $NH_2$, $NH(C_1-C_4)$-Alkyl, $N[(C_1-C_4)\text{-Alkyl}]_2$, $(C_2-C_4)$-Alkenyl, $(C_2-C_4)$-Alkinyl, $(C_2-C_4)$-Alkenyloxy oder $(C_2-C_4)$-Alkinyloxy,

$R_9$ Wasserstoff oder $(C_1-C_4)$-Alkyl,

$R_{10}$ $(C_1-C_4)$-Alkyl oder Benzyl

X CH, C-Hal mit Hal = Chlor oder Brom, $C-(C_1-C_4)$-Alkyl, $C-(C_1-C_4)$-Alkoxy oder N,

m eine Zahl von null bis drei und

n eine Zahl von null bis zwei

bedeuten.

2. Verfahren zur Herstellung von Verbindungen der Formel I, von Anspruch 1, dadurch gekennzeichnet, daß man Verbindungen der Formel II, worin X' = Chlor oder Fluor bedeutet und

$X' - SO_2 - NHCO - N - R_6$ (II)
with $R_5$ on the N

$R_5$, $R_6$ die Bedeutungen wie in Formel I besitzen, mit Enaminen der Formel III, worin $R_1$ bis $R_4$

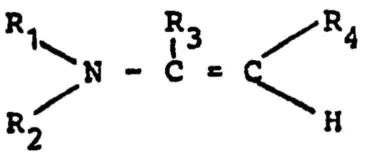
III

die Bedeutungen wie in Formel I besitzen, umsetzt.

**Patentanspruch** für den Vertragsstaat AT

Verfahren zur Herstellung von Verbindungen der Formel I, worin

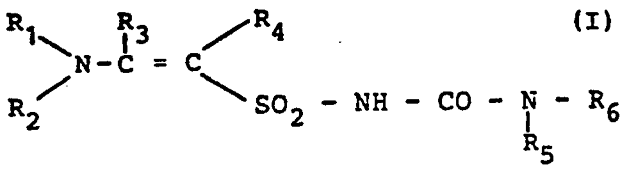
(I)

$R_1$ und $R_2$ unabhängig voneinander Wasserstoff, $(C_1-C_8)$-Alkyl, $(C_3-C_7)$-Cycloalkyl, Phenyl, welches gegebenenfalls ein- oder mehrfach durch Halogen, $(C_1-C_4)$-Alkyl, $(C_1-C_4)$-Alkoxy, $(C_1-C_4)$-Alkylthio, $(C_1-C_4)$-Alkoxycarbonyl, $CF_3$, $NO_2$ oder $CH_3SO_2$-substituiert sein kann, bedeuten oder die Reste $R_1$ und $R_2$ zusammen mit dem gemeinsamen Stickstoffatom einen 5- bis 7-gliedrigen aliphatischen Ring bilden, worin eine $CH_2$-Grup-pe gegebenenfalls durch Sauerstoff, NH oder $N(C_1-C_4)$-Alkyl ersetzt sein kann,

$R_3$ Wasserstoff, $(C_3-C_7)$Cycloalkyl, $(C_1-C_6)$Alkyl, $(C_1-C_4)$-Alkoxy, $(C_2-C_4)$Alkenyloxy, $(C_2-C_4)$Alkinyloxy, $(C_1-C_4)$Alkylsulfenyl, $(C_1-C_4)$Alkylsulfinyl, $(C_1-C_4)$-Alkylsulfonyl, $-SO_2NH(C_1-C_4)$Alkyl, $-SO_2N[(C_1-C_4)\text{-Alkyl}]_2$, einen Phenylrest, der gegebenenfalls ein- oder mehrfach durch Halogen, $CF_3$, $(C_1-C_4)$Alkyl, $(C_1-C_4)$Alkoxy, $(C_1-C_4)$Alkoxycarbonyl, $(C_1-C_4)$Alkylsulfenyl, $(C_1-C_4)$Alkylsulfinyl oder $(C_1-C_4)$Alkylsul-fonyl substituiert ist, Benzyl, $(C_1-C_4)$Alkoxycarbonyl, $(C_2-C_4)$Alkenyloxycarbonyl, $(C_2-C_4)$Alkinyloxycarbonyl, Aminocarbonyl, $(C_1-C_4)$Alkylaminocarbonyl oder $[(C_1-C_4)\text{Alkyl}]_2$aminocarbonyl,

$R_4$ die Bedeutung von $R_3$ umfaßt und zusätzlich Formyl, $(C_1-C_6)$-Alkanoyl, Benzoyl, das gegebenenfalls durch Halogen, $(C_1-C_4)$Alkyl, $(C_1-C_4)$Alkoxy, $(C_1-C_4)$Alkylthio, $CF_3$, $NO_2$ oder $(C_1-C_4)$Alkoxycarbonyl substituiert sein kann, $(C_3-C_7)$Cycloalkylcarbonyl, $C_6H_5-CH_2-O-CO-$, bedeuten oder $R_3$ und $R_4$ gemeinsam mit den sie verbindenden olefinischen Kohlenstoffatomen ein 5- bis 8-gliedriges cycloaliphatisches Ringsystem bilden,

$R_5$ Wasserstoff, $(C_1-C_6)$-Alkyl, $(C_1-C_4)$-Alkenyl, $(C_1-C_4)$-Alkinyl, $(C_1-C_4)$-Alkoxy oder $(C_1-C_4)$-Alkoxycarbonyl bedeutet,

$R_6$ $(C_1-C_8)$-Alkyl oder $(C_3-C_7)$-Cycloalkyl, Phenyl, welches gegebenenfalls ein oder mehrfach durch Halogen, $(C_1-C_4)$-Alkyl, $(C_1-C_4)$-Alkoxy, $(C_1-C_4)$-Alkylthio, $CF_3$, $NO_2$ oder $(C_1-C_4)$-Alkoxycarbonyl substituiert sein kann oder den Rest

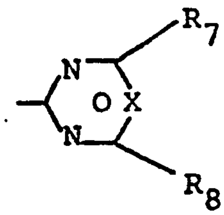

bedeutet, worin

$R_7$ und $R_8$ unabhängig voneinander Wasserstoff, $(C_1-C_4)$-Alkyl oder $(C_1-C_4)$-Alkoxy, welche gegebenenfalls ein- bis zweifach durch $(C_1-C_2)$-Alkoxy oder ein- bis dreifach durch Halogen substituiert sind, Halogen, $-(CHR_9)_m-S(O)_nR_{10}$, $NH_2$, $NH(C_1-C_4)$-Alkyl, $N[(C_1-C_4)\text{-Alkyl}]_2$, $(C_2-C_4)$-Alkenyl, $(C_2-C_4)$-Alkinyl, $(C_2-C_4)$-Alkenyloxy oder $(C_2-C_4)$-Alkinyloxy,

$R_9$ Wasserstoff oder $(C_1-C_4)$-Alkyl,

$R_{10}$ $(C_1-C_4)$-Alkyl oder Benzyl

X CH, C-Hal mit Hal = Chlor oder Brom, $C-(C_1-C_4)$-Alkyl, $C-(C_1-C_4)$-Alkoxy oder N,

m eine Zahl von null bis drei und

n eine Zahl von null bis zwei

bedeuten

dadurch geekennzeichnet daß man Verbindungen der Formel II, worin X' = Chlor oder Fluor bedeutet und

$X' - SO_2 - NHCO - N - R_6$ (II)
with $R_5$ on the N

$R_5$, $R_6$ die Bedeutungen wie in Formel I besitzen, mit Enaminen der Formel III, worin $R_1$ bis $R_4$

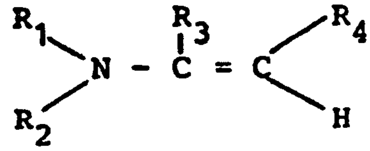
III

die Bedeutungen wie in Formel I besitzen, umsetzt.

**Claims** for the Contracting States: BE, CH, DE, FR, GB, IT, LI, NL, SE

1. A compound of the formula I

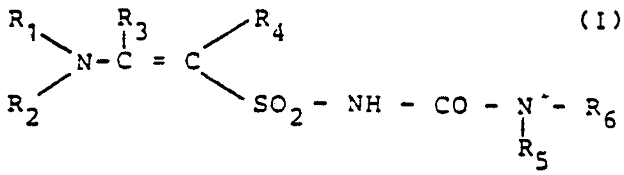

(I)

in which

$R_1$ and $R_2$ independently of one another denote hydrogen, $(C_1$-$C_8)$-alkyl, $(C_3$-$C_7)$-cycloalkyl, or phenyl, which can optionally be monosubstituted or polysubstituted by halogen, $(C_1$-$C_4)$-alkyl, $(C_1$-$C_4)$-alkoxy, $(C_1$-$C_4)$-alkylthio, $(C_1$-$C_4)$-alkoxycarbonyl, $CF_3$, $NO_2$ or $CH_3SO_2$-, or the radicals $R_1$ and $R_2$, together with the common nitrogen atom, form a 5-membered to 7-membered aliphatic ring, in which one $CH_2$ group can optionally be replaced by oxygen, NH or $N(C_1$-$C_4)$-alkyl,

$R_3$ denotes hydrogen, $(C_3$-$C_7)$cycloalkyl, $(C_1$-C6)alkyl, $(C_1$-$C_4)$alkoxy, $(C_2$-$C_4)$alkenyloxy, $(C_2$-$C_4)$alkynyloxy, $(C_1$-$C_4)$alkylsulfenyl, $(C_1$-$C_4)$alkylsulfinyl, $(C_1$-$C_4)$-alkylsulfonyl, $-SO_2NH(C_1$-$C_4)$alkyl, $-SO_2N((C_1$-$C_4)$-alkyl$]_2$, a phenyl radical which is optionally monosubstituted or polysubstituted by halogen, $CF_3$, $(C_1$-$C_4)$alkyl, $(C_1$-$C_4)$alkoxy, $(C_1$-$C_4)$alkoxycarbonyl, $(C_1$-$C_4)$alkylsulfenyl, $(C_1$-$C_4)$alkylsulfinyl or $(C_1$-$C_4)$-alkylsulfonyl, or benzyl, $(C_1$-$C_4)$-alkoxycarbonyl, $(C_2$-$C_4)$alkenyloxycarbonyl, $(C_2$-$C_4)$alkynyloxycarbonyl, aminocarbonyl, $(C_1$-$C_4)$-alkylaminocarbonyl or $[(C_1$-$C_4)$alkyl$]_2$aminocarbonyl,

$R_4$ has the meaning of $R_3$ and additionally denotes formyl, $(C_1$-$C_6)$-alkanoyl, benzoyl which can optionally be substituted by halogen, $(C_1$-$C_4)$-alkyl, $(C_1$-$C_4)$alkoxy, $(C_1$-$C_4)$alkylthio, $CF_3$, $NO_2$ or $(C_1$-$C_4)$alkoxycarbonyl, or $(C_3$-C7)-cycloalkylcarbonyl or $C_6H_5$-$CH_2$-O-CO-, or $R_3$ and $R_4$ together with the olefinic carbon atoms connecting them form a 5-membered to 8-membered cycloaliphatic ring system,

$R_5$ denotes hydrogen, $(C_1$-C6)-alkyl, $(C_1$-$C_4)$-alkenyl, $(C_1$-$C_4)$-alkynyl, $(C_1$-$C_4)$-alkoxy or $(C_1$-$C_4)$-alkoxycarbonyl,

$R_6$ denotes $(C_1$-$C_8)$-alkyl or $(C_3$-$C_7)$-cycloalkyl, phenyl which can optionally be monosubstituted or polysubstituted by halogen, $(C_1$-$C_4)$-alkyl, $(C_1$-$C_4)$-alkoxy, $(C_1$-$C_4)$-alkylthio, $CF_3$, $NO_2$ or $(C_1$-$C_4)$-alkoxycarbonyl, or the radical

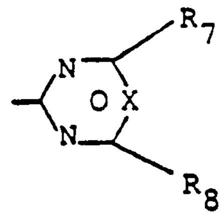

in which

$R_7$ and $R_8$ independently of one another denote hydrogen, $(C_1$-$C_4)$-alkyl or $(C_1$-$C_4)$-alkoxy, which are optionally monosubstituted or disubstituted by $(C_1$-$C_2)$-alkoxy or mono-, di- or tri-substituted by halogen, or halogen, $-(CHR_9)_m$-$S(O)_nR_{10}$, $NH_2$, $NH(C_1$-$C_4)$-alkyl, $N[(C_1$-$C_4)$-alkyl$]_2$, $(C_2$-$C_4)$-alkenyl, $(C_2$-$C_4)$-alkynyl, $(C_2$-$C_4)$-alkenyloxy or $(C_2$-$C_4)$-alkynyloxy,

$R_9$ denotes hydrogen or $(C_1$-$C_4)$-alkyl,

$R_{10}$ denotes $(C_1$-$C_4)$-alkyl or benzyl,

X denotes CH, C-Hal, where Hal = chlorine or bromine, C-$(C_1$-$C_4)$-alkyl, C-$(C_1$-$C_4)$-alkoxy or N,

m denotes a number from zero to three and

n denotes a number from zero to two.

2. A process for the preparation of a compound of the formula I as claimed in claim 1, which comprises reacting a compound of the formula II

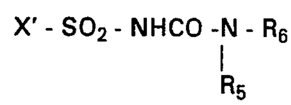

(II)

in which X' denotes chlorine or fluorine and $R_5$ and $R_6$ have the meanings as in formula I, with enamines of the formula III

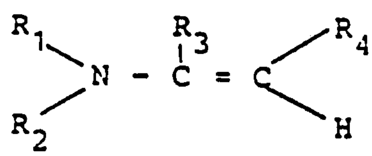

III

in which $R_1$ to $R_4$ have the meanings as in formula I.

**Claim** for the contracting state AT

A process for the preparation of a compound of the formula I

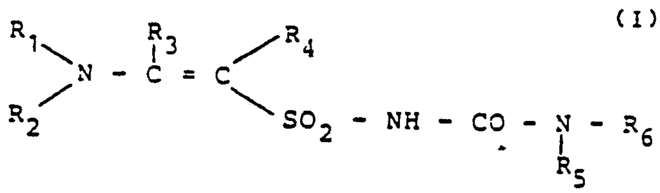

(I)

in which

$R_1$ and $R_2$ independently of one another denote hydrogen, $(C_1$-$C_8)$-alkyl, $(C_3$-$C_7)$-cycloalkyl, or

phenyl, which can optionally be monosubstituted or polysubstituted by halogen, $(C_1-C_4)$-alkyl, $(C_1-C_4)$-alkoxy, $(C_1-C_4)$-alkylthio, $(C_1-C_4)$-alkoxycarbonyl, $CF_3$, $NO_2$ or $CH_3SO_2$-, or the radicals $R_1$ and $R_2$, together with the common nitrogen atom, form a 5-membered to 7-membered aliphatic ring, in which one $CH_2$ group can optionally be replaced by oxygen, NH or $N(C_1-C_4)$-alkyl,

$R_3$ denotes hydrogen, $(C_3-C_7)$cycloalkyl, $(C_1-C_6)$alkyl, $(C_1-C_4)$alkoxy, $(C_2-C_4)$alkenyloxy, $(C_2-C_4)$alkynyloxy, $(C_1-C_4)$alkylsulfenyl, $(C_1-C_4)$alkylsulfinyl, $(C_1-C_4)$-alkylsulfonyl, $-SO_2NH(C_1-C_4)$alkyl, $-SO_2N[(C_1-C_4)\text{-alkyl}]_2$, a phenyl radical which is optionally monosubstituted or polysubstituted by halogen, $CF_3$, $(C_1-C_4)$alkyl, $(C_1-C_4)$alkoxy, $(C_1-C_4)$alkoxycarbonyl, $(C_1-C_4)$alkylsulfenyl, $(C_1-C_4)$alkylsulfinyl or $(C_1-C_4)$-alkylsulfonyl, or benzyl, $(C_1-C_4)$-alkoxycarbonyl, $(C_2-C_4)$alkenyloxycarbonyl, $(C_2-C_4)$alkynyloxycarbonyl, aminocarbonyl, $(C_1-C_4)$-alkylaminocarbonyl or $[(C_1-C_4)alkyl]_2$aminocarbonyl,

$R_4$ has the meaning of $R_3$ and additionally denotes formyl, $(C_1-C_6)$-alkanoyl, benzoyl which can optionally be substituted by halogen, $(C_1-C_4)$-alkyl, $(C_1-C_4)$alkoxy, $(C_1-C_4)$alkylthio, $CF_3$, $NO_2$ or $(C_1-C_4)$alkoxycarbonyl, or $(C_3-C_7)$-cycloalkylcarbonyl or $C_6H_5-CH_2-O-CO-$, or $R_3$ and $R_4$ together with the olefinic carbon atoms connecting them form a 5-membered to 8-membered cycloaliphatic ring system,

$R_5$ denotes hydrogen, $(C_1-C_6)$-alkyl, $(C_1-C_4)$-alkenyl, $(C_1-C_4)$-alkynyl, $(C_1-C_4)$-alkoxy or $(C_1-C_4)$-alkoxycarbonyl,

$R_6$ denotes $(C_1-C_8)$-alkyl or $(C_3-C_7)$-cycloalkyl, phenyl which can optionally be monosubstituted or polysubstituted by halogen, $(C_1-C_4)$-alkyl, $(C_1-C_4)$-alkoxy, $(C_1-C_4)$-alkylthio, $CF_3$, $NO_2$ or $(C_1-C_4)$-alkoxycarbonyl, or the radical

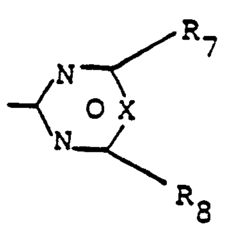

in which

$R_7$ and $R_8$ independently of one another denote hydrogen, $(C_1-C_4)$-alkyl or $(C_1-C_4)$-alkoxy, which are optionally monosubstituted or disubstituted by $(C_1-C_2)$-alkoxy or mono-, di- or tri-substituted by halogen, or halogen, $-(CHR_9)_m-S(O)_nR_{10}$, $NH_2$, $NH(C_1-C_4)$-alkyl, $N[(C_1-C_4)\text{-alkyl}]_2$, $(C_2-C_4)$-alkenyl, $(C_2-C_4)$-alkynyl, $(C_2-C_4)$-alkenyloxy or $(C_2-C_4)$-alkynyloxy,

$R_9$ denotes hydrogen or $(C_1-C_4)$-alkyl,

$R_{10}$ denotes $(C_1-C_4)$-alkyl or benzyl,

X denotes CH, C-Hal, where Hal = chlorine or bromine, $C-(C_1-C_4)$-alkyl, $C-(C_1-C_4)$-alkoxy or N,

m denotes a number from zero to three and

n denotes a number from zero to two, which comprises reacting a compound of the formula II

$X' - SO_2 - NHCO - N - R_6$ (II)
|
$R_5$

in which X' denotes chlorine or fluorine and $R_5$ and $R_6$ have the meanings as in formula I, with enamines of the formula III

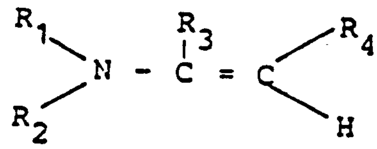

III

in which $R_1$ to $R_4$ have the meanings as in formula I.

**Revendications** pour les Etats contractants: BE, CH, DE, FR, GB, IT, LI, NL, SE

1. Composés répondant à la formule I:

(I)

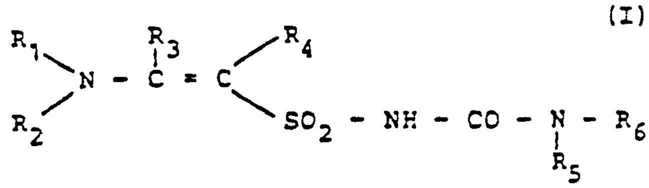

dans laquelle

$R_1$ et $R_2$ représentent chacun, indépendamment l'un de l'autre, l'hydrogène, un alkyle en $C_1-C_8$, un cycloalkyle en $C_3-C_7$, un phényle éventuellement porteur d'un ou de plusieurs substituants pris dans l'ensemble constitué par les halogènes, les alkyles en $C_1-C_4$, les alcoxy en $C_1-C_4$, les alkylthio en $C_1-C_4$, les $(C_1-C_4)$alcoxy-carbonyles, $CF_3$, $NO_2$ et $CH_3SO_2$-, ou $R_1$ et $R_2$ forment ensemble et avec l'atome d'azote qui les porte un cycle aliphatique comportant de 5 à 7 maillons et dans lequel un radical $CH_2$ peut éventuellement être remplacé par un atome d'oxygène, un radical NH ou un radical $N(C_1-C_4)$alkyl,

$R_3$ représente l'hydrogène, un cycloalkyle en $C_3-C_7$, un alkyle en $C_1-C_6$, un alcoxy en $C_1-C_4$, un alcényloxy en $C_2-C_4$, un alcynyloxy en $C_2-C_4$, un alkylsulfényle en $C_1-C_4$, un alkylsulfinyle en $C_1-C_4$, un alkylsulfonyle en $C_1-C_4$, un radical $-SO_2NH(C_1-C_4)$alkyl, un radical $-SO_2N[(C_1-C_4)alkyl]_2$, un radical phényle éventuellement porteur d'un ou de plusieurs substituants pris dans l'ensemble constitué par les halogènes, $CF_3$, les alkyles en $C_1-C_4$, les alcoxy en $C_1-C_4$, les $(C_1-C_4)$alcoxy-carbonyles, les alkylsulfényles en $C_1-C_4$, les alkylsulfinyles en $C_1-C_4$ et les alkylsulfonyles en $C_1-C_4$, un benzyle, un $(C_1-C_4)$alcoxy-carbonyle, un $(C_2-C_4)$alcényloxy-carbonyle, un $(C_2-C_4)$alcynyloxy-carbonyle, un aminocarbonyle, un $(C_1-C_4)$alkylamino-carbonyle ou un $[(C_1-C_4)alkyl]_2$

amino-carbonyle,

$R_4$ a la signification de $R_3$ et peut en outre représenter un formyle, un alcanoyle en $C_1$-$C_6$, un benzoyle éventuellement porteur d'un halogène, d'un alkyle en $C_1$-$C_4$, d'un alcoxy en $C_1$-$C_4$, d'un alkylthio en $C_1$-$C_4$, d'un $CF_3$, d'un $NO_2$ ou d'un ($C_1$-$C_4$)alcoxy-carbonyle, un ($C_3$-$C_7$)cycloalkyl-carbonyle ou un radical $C_6H_5$-$CH_2$-O-CO-, ou

$R_3$ et $R_4$ forment ensemble, et avec les atomes de carbone éthyléniques qui les unissent, un système cyclo-aliphatique comportant de 5 à 8 maillons,

$R_5$ représente l'hydrogène, un alkyle en $C_1$-$C_6$, un alcényle en $C_1$-$C_4$, un alcynyle en $C_1$-$C_4$, un alcoxy en $C_1$-$C_4$ ou un ($C_1$-$C_4$)alcoxy-carbonyle, et

$R_6$ représente un alkyle en $C_1$-$C_6$, un cycloalkyle en $C_3$-$C_7$, un phényle éventuellement porteur d'un ou de plusieurs substituants pris parmi les halogènes, les alkyles en $C_1$-$C_4$, les alcoxy en $C_1$-$C_4$, les alkylthio en $C_1$-$C_4$, $CF_3$, $NO_2$ et les ($C_1$-$C_4$)alcoxy-carbonyles, ou un radical de formule:

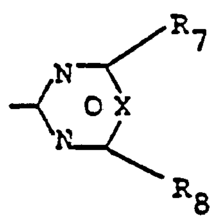

dans lequel

$R_7$ et $R_8$ représentent chacun, indépendamment l'un de l'autre, l'hydrogène, un alkyle en $C_1$-$C_4$ ou un alcoxy en $C_1$-$C_4$ éventuellement porteurs d'un ou de deux alcoxy en $C_1$ ou $C_2$ ou d'un à trois halogènes, un halogène ou un radical $-(CHR_9)_m-S(O)_nR_{10}$, $NH_2$, $NH(C_1$-$C_4)$alkyl, $N[(C_1$-$C_4)alkyl]_2$, alcényle en $C_2$-$C_4$, alcynyle en $C_2$-$C_4$, alcényloxy en $C_2$-$C_4$ ou alcynyloxy en $C_2$-$C_4$,

$R_9$ représente l'hydrogène ou un alkyle en $C_1$-$C_4$,

$R_{10}$ représente un alkyle en $C_1$-$C_4$ ou un benzyle,

X représente CH C-Hal (Hal désignant le chlore ou le brome), C-($C_1$-$C_4$)alkyl, C-($C_1$-$C_4$)alcoxy ou N,

m désigne un nombre de 0 à 3 et

n désigne un nombre de 0 à 2.

2. Procédé de préparation de composés de formule I selon la revendication 1, procédé caractérisé en ce qu'on fait réagir des composés répondant à la formule II:

$$X'\text{-} SO_2 \text{-} NHCO \text{-} \underset{\displaystyle R_5}{\underset{|}{N}} \text{-} R_6 \qquad (II)$$

dans laquelle X' représente le chlore ou le fluor, et $R_5$ et $R_6$ ont les significations qui leur ont été données à propos de la formule I, avec des énamines répondant à la formule III:

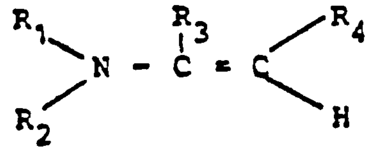

III

dans laquelle $R_1$ à $R_4$ ont les significations qui leur ont été données à propos de la formule I.

**Revendication** pour l'Etat contractant: AT

Procédé de préparation de composés répondant à la formule I:

(I)

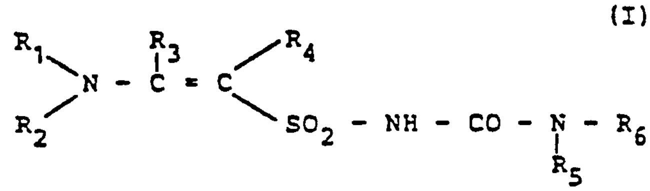

dans laquelle

$R_1$ et $R_2$ représentent chacun, indépendamment l'un de l'autre, l'hydrogène, un alkyle en $C_1$-$C_8$, un cycloalkyle en $C_3$-$C_7$, un phényle éventuellement porteur d'un ou de plusieurs substituants pris dans l'ensemble constitué par les halogènes, les alkyles en $C_1$-$C_4$, les alcoxy en $C_1$-$C_4$, les alkylthio en $C_1$-$C_4$, les ($C_1$-$C_4$)alcoxy-carbonyles, $CF_3$, $NO_2$ et $CH_3SO_2$-, ou $R_1$ et $R_2$ forment ensemble et avec l'atome d'azote qui les porte un cycle aliphatique comportant de 5 à 7 maillons et dans lequel un radical $CH_2$ peut éventuellement être remplacé par un atome d'oxygène, un radical NH ou un radical $N(C_1$-$C_4)$alkyl,

$R_3$ représente l'hydrogène, un cycloalkyle en $C_3$-$C_7$, un alkyle en $C_1$-$C_6$, un alcoxy en $C_1$-$C_4$, un alcényloxy en $C_2$-$C_4$, un alcynyloxy en $C_2$-$C_4$, un alkylsulfényle en $C_1$-$C_4$, un alkylsulfinyle en $C_1$-$C_4$, un alkylsulfonyle en $C_1$-$C_4$, un radical $-SO_2NH(C_1$-$C_4)$alkyl, un radical $-SO_2N[(C_1$-$C_4)alkyl]_2$, un radical phényle éventuellement porteur d'un ou de plusieurs substituants pris dans l'ensemble constitué par les halogènes, $CF_3$, les alkyles en $C_1$-$C_4$, les alcoxy en $C_1$-$C_4$, les ($C_1$-$C_4$)alcoxy-carbonyles, les alkylsulfényles en $C_1$-$C_4$, les alkylsulfinyles en $C_1$-$C_4$ et les alkylsulfonyles en $C_1$-$C_4$, un benzyle, un ($C_1$-$C_4$)alcoxy-carbonyle, un ($C_2$-$C_4$)alcényloxy-carbonyle, un ($C_2$-$C_4$)alcynyloxy-carbonyle, un aminocarbonyle, un ($C_1$-$C_4$)alkylamino-carbonyle ou un $[(C_1$-$C_4)alkyl]_2$ amino-carbonyle,

$R_4$ a la signification de $R_3$ et peut en outre représenter un formyle, un alcanoyle en $C_1$-$C_6$, un benzoyle éventuellement porteur d'un halogène, d'un alkyle en $C_1$-$C_4$, d'un alcoxy en $C_1$-$C_4$, d'un

alkylthio en $C_1$-$C_4$, d'un $CF_3$, d'un $NO_2$ ou d'un ($C_1$-$C_4$)alcoxy-carbonyle, un ($C_3$-$C_7$)cycloalkyl-carbonyle ou un radical $C_6H_5$-$CH_2$-O-CO-, ou

$R_3$ et $R_4$ forment ensemble, et avec les atomes de carbone éthyléniques qui les unissent, un système cyclo-aliphatique comportant de 5 à 8 maillons,

$R_5$ représente l'hydrogène, un alkyle en $C_1$-$C_6$, un alcényle en $C_1$-$C_4$, un alcynyle en $C_1$-$C_4$, un alcoxy en $C_1$-$C_4$ ou un ($C_1$-$C_4$)alcoxy-carbonyle, et

$R_6$ représente un alkyle en $C_1$-$C_8$, un cycloalkyle en $C_3$-$C_7$, un phényle éventuellement porteur d'un ou de plusieurs substituants pris parmi les halogènes, les alkyles en $C_1$-$C_4$, les alcoxy en $C_1$-$C_4$, les alkylthio en $C_1$-$C_4$, $CF_3$, $NO_2$ et les ($C_1$-$C_4$)alcoxy-carbonyles, ou un radical de formule:

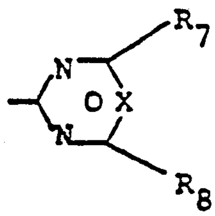

dans lequel

$R_7$ et $R_6$ représentent chacun, indépendamment l'un de l'autre, l'hydrogène, un alkyle en $C_1$-$C_4$ ou un alcoxy en $C_1$-$C_4$ éventuellement porteurs d'un ou de deux alcoxy en $C_1$ ou $C_2$ ou d'un à trois halogènes, un halogène ou un radical $-(CHR_9)_m-S(O)_nR_{10}$, $NH_2$, $NH(C_1$-$C_4)alkyl$, $N[(C_1$-$C_4)alkyl]_2$, alcényle en $C_2$-$C_4$, alcynyle en $C_2$-$C_4$, alcényloxy en $C_2$-$C_4$ ou alcynyloxy en $C_2$-$C_4$,

$R_9$ représente l'hydrogène ou un alkyle en $C_1$-$C_4$,

$R_{10}$ représente un alkyle en $C_1$-$C_4$ ou un benzyle,

X représente CH, C-Hal (Hal désignant le chlore ou le brome), C-($C_1$-$C_4$)alkyl, C-($C_1$-$C_4$)alcoxy ou N,

m désigne un nombre de 0 à 3 et

n désigne un nombre de 0 à 2,

procédé caractérisé en ce qu'on fait réagir des composés répondant à la formule II:

$$X'-SO_2-NHCO-N(R_5)-R_6 \qquad (II)$$

dans laquelle X' représente le chlore ou le fluor, et $R_5$ et $R_6$ ont les significations qui leur ont été données à propos de la formule I, avec des énamines répondant à la formule III:

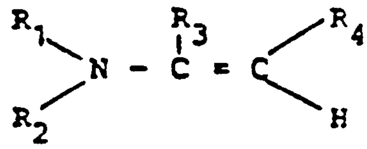

III

dans laquelle $R_1$ à $R_4$ ont les significations qui leur ont été données à propos de la formule I.